# EUROPEAN PATENT APPLICATION

(11) **EP 3 903 723 A1**
(43) Date of publication of application: **03.11.2021**
(21) Application number: 19905754.8
(22) Date of filing: 06.05.2019
(51) Int. Cl.: A61C 1/00, A61C 1/08

(54) **DENTAL IMPLANT HANDSET**

(30) Priority: 28.12.2018 CN 201822230938 U
(71) Applicant: Beijing Yakebot Technology Co., Ltd, Beijing 100744 (CN)
(72) Inventor: WANG, Lifeng, Beijing 100007 (CN)
(74) Representative: Naeven, Ralf
(86) International application number: PCT/CN2019/085673
(87) International publication number: WO 2020/133867

(57) **Abstract**

Disclosed is a dental implant handpiece, comprising an implant handpiece body (1), and a drill bit (2) disposed on the implant handpiece body (1). The implant handpiece body (1) is further provided with an image collector disposed close to the drill bit (2) and configured to acquire images of locations in the vicinity of the drill bit (2) in an oral cavity. In a whole treatment process, a dentist is free of double-handed operation and only needs to hold the dental implant handpiece with one hand. During the treatment process, images near the drill bit (2) are acquired in real time, thus the treatment situation is controlled in real time when each treatment action is executed, and the position and angle of the drill bit (2) may be adjusted at any time according to the images acquired by the image collector.

## Description

### CROSS-REFERENCE TO RELATED APPLICATION

The present application claims priority to Chinese Application No. 2018222309389 filed on December 28, 2018, entitled "Dental Implant Handpiece", which is hereby incorporated by reference in its entirety.

### FIELD OF TECHNOLOGY

The present application relates to the technical field of medical devices, and in particular to a dental implant handpiece.

### BACKGROUND

A dental handpiece is a clinical tool used for cutting, drilling and polishing teeth. Currently, when treating a patient with a dental handpiece, a dentist has to hold the dental handpiece in one hand to drill and polish the patient's teeth, and hold a stomatoscope in the other hand to keep an eye on the treatment. The dentist needs to use the stomatoscope for observation every time a treatment action is completed, which not only brings inconvenience to the dentist, but also brings discomfort to the patient due to frequent use of the stomatoscope.

### BRIEF SUMMARY

An objective of the present application is to solve the technical problem that the traditional dental handpiece needs to be additionally equipped with a stomatoscope for auxiliary observation during the treatment process, which causes inconvenience in operation.

In order to solve the above-mentioned problem, the present application provides a dental implant handpiece, including an implant handpiece body and a drill bit disposed on the implant handpiece body; wherein the implant handpiece body is further provided with an image collector disposed close to the drill bit and configured to acquire images of areas in the vicinity of the drill bit in an oral cavity.

In an embodiment, the image collector is disposed in an inner cavity of the implant handpiece body, and the implant handpiece body is provided with a lens hole and a connection hole at two ends of the implant handpiece body, respectively; the image collector acquires images through the lens hole, and the image collector is connected to an external display device through the connection hole.

In an embodiment, the inner cavity of the implant handpiece body is provided with a drive bevel gear, a driven bevel gear and a reducer, a socket is provided on a side wall of a top end of the implant handpiece body, a first end of the drill bit is inserted in the socket and a second end of the drill bit extends outward; the driven bevel gear is disposed at the first end of the drill bit, the drive bevel gear meshes with the driven bevel gear, and the drive bevel gear is connected to the reducer through a transmission shaft.

In an embodiment, the reducer includes a motor, an inner ring gear, a planet carrier, and a planet gear and a sun gear disposed in the inner ring gear; the transmission shaft is connected to the planet gear through the planet carrier, the planet gear is located between the inner ring gear and the sun gear, and the planet gear meshes with the inner ring gear and the sun gear, respectively; and the sun gear is connected to the motor through a rotating shaft.

In an embodiment, the implant handpiece body includes a handle and a head, one end of the head is connected to the handle, and the other end of the head is provided with the drill bit; and an axis of the drill bit is perpendicular to an axis of the head.

In an embodiment, the axis of the head is collinear with an axis of the handle.

In an embodiment, the image collector includes an endoscope and an optical fiber, one end of the optical fiber is connected to the endoscope, and the other end of the optical fiber is configured to be connected to an external display device.

In an embodiment, the endoscope is an optical endoscope, fiber endoscope, electronic endoscope, CCD video endoscope or CMOS video endoscope.

In an embodiment, the image collector includes a light source and a camera, and the light source is disposed close to the camera and configured to illuminate a shooting area of the camera.

In an embodiment, a resolution increasing button and a resolution decreasing button respectively electrically connected to the camera are provided on a side wall of the implant handpiece body.

The dental implant handpiece of the present application is simple in structure and convenient in operation. During the whole treatment process, a dentist is free of double-handed operation and only needs to hold the dental implant handpiece with one hand. In addition, compared to the existing dental handpieces with which a dentist may only observe the treatment situation with the help of a stomatoscope after a treatment action is completed. The dental implant handpiece provided by the present application may acquire images of the vicinity of the drill bit in real time during the treatment, so that the dentist can not only control the treatment situation in real time when performing each treatment action, but also adjust the position and angle of the drill bit at any time according to the images acquired by the image collector. Therefore, not only may the precision and effectiveness of treatment be significantly improved, but also patient's comfort may be enhanced.

### BRIEF DESCRIPTION OF THE DRAWINGS

FIG. 1 is a longitudinal cross-sectional view of a dental implant handpiece during treatment according to an embodiment of the present application;
FIG. 2 is a perspective diagram of a dental implant handpiece according to an embodiment of the present application;
FIG. 3 is a longitudinal cross-sectional view of FIG. 2;
FIG. 4 is a longitudinal cross-sectional view of a dental implant handpiece according to an embodiment of the present application;
FIG. 5 is a partial cross-sectional view of a dental implant handpiece with a hidden drill bit according to an embodiment of the present application;
FIG. 6 is a partial cross-sectional view of a dental implant handpiece according to an embodiment of the present application; and
FIG. 7 is a rear view of a dental implant handpiece according to an embodiment of the present application.

### Reference numerals:

| | |
|---|---|
| 1 implant handpiece body | 1-1 lens hole |
| 1-2 connection hole | 1-3 head |
| 1-4 handle | 2 drill bit |
| 3-1 endoscope | 3-2 optical fiber |
| 4-1 drive bevel gear | 4-2 driven bevel gear |
| 4-3 transmission shaft | 5-1 inner ring gear |
| 5-2 sun gear | 5-3 planet gear |
| 5-4 planet carrier | 5-5 rotating shaft |

### DETAILED DESCRIPTION

In order to illustrate the objectives, technical solutions and advantages of the present application more clearly, the technical solutions in the present application will be described clearly in conjunction with the accompanying drawings in the present application. Obviously, the described embodiments are part of the embodiments of the present application, rather than all of the embodiments. All other embodiments obtained by a person of ordinary skill in the art based on the embodiments of the present application without any creative effort fall within the protection scope of the present application.

In the description of the present application, unless otherwise stated, the orientations or positional relationships indicated by terms such as "upper" and "lower" are based on the orientation or positional relationship shown in the drawings, and are intended only to facilitate the description of the present application and simplify the description, rather than to indicate or imply that a device or component referred to must have a particular orientation, or be constructed and operated in a particular orientation, and thus can not be construed as limiting the present application.

It should be noted that unless otherwise clearly specified and defined, the terms "connected" shall be understood broadly, for example, it may be either fixedly connected or detachably connected, or may be integrally connected; it may be directly connected, or indirectly connected through an intermediary. The specific meanings of the terms above in the present application can be understood by a person skilled in the art in accordance with specific conditions.

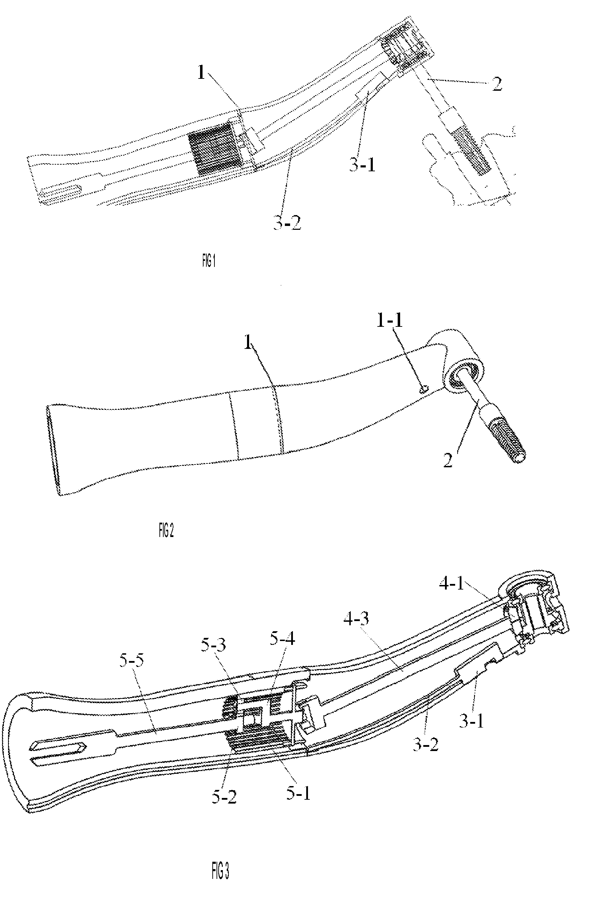

As shown in FIGS. 1 and 2, an embodiment provides a dental implant handpiece, including an implant handpiece body 1 and a drill bit 2 disposed on the implant handpiece body 1; the implantation handpiece body 1 is further provided with an image collector disposed close to the drill bit 2 and configured to acquire images of areas in the vicinity of the drill bit 2 in an oral cavity. Before a treatment, the image collector is connected to an external display device. The implant handpiece body 1 is provided with an image collector, that is, the dental implant handpiece in this embodiment comes with an image collector, thus when a dentist holds the implant handpiece body 1 to drill and polish a patient's teeth, the image collector will transmit images of the vicinity of the drill bit 2 to the external display in real time, so that the dentist can not only control the treatment situation in real time when performing each treatment action, but also adjust the position and angle of the drill bit 2 at any time according to the images acquired by the image collector.

It can be seen that in the whole treatment process, the dentist is free of double-handed operation and only needs to hold the dental implant handpiece with one hand. In addition, compared to the existing dental handpieces with which a dentist can only observe the treatment situation after each treatment action is completed with the help of a stomatoscope, the dental implant handpiece provided by the this embodiment may acquire images of the vicinity of the drill bit 2 in real time during the treatment, so that the dentist may not only control the treatment situation in real time when performing each treatment action, but also adjust the position and angle of the drill bit 2 at any time according to the images acquired by the image collector. Therefore, not only can the precision and effectiveness of treatment be significantly improved, but also patient's comfort can be enhanced.

In an embodiment, the image collector includes an endoscope 3-1 and an optical fiber 3-2. One end of the optical fiber 3-2 is connected to the endoscope 3-1, and the other end of the optical fiber 3-2 is configured to be connected to an external display device, wherein the endoscope 3-1 is an optical endoscope, fiber endoscope, electronic endoscope, CCD video endoscope or CMOS video endoscope. It should be noted that in addition to an endoscope 3-1 and an optical fiber 3-2, the image collector may also include a light source and a camera, wherein the light source is disposed close to the camera and configured to illuminate a shooting area of the camera. Further, in order to ensure that the images captured by the camera are clear and complete, a resolution increasing button and a resolution decreasing button respectively electrically connected to the camera are provided on a side wall of the implant handpiece body 1. During treatment, the dentist may adjust the resolution of the camera by pressing the resolution increasing button or the resolution decreasing button, thereby changing the definition of the images captured by the camera.

In an embodiment, the image collector is disposed in an inner cavity of the implant handpiece body 1, and the implant handpiece body 1 is provided with a lens hole 1-1 and a connection hole 1-2 at two ends of the implant handpiece body 1, respectively; the image collector acquires images through the lens hole 1-1, and the image collector is connected to an external display device through the connection hole 1-2. Of course, in order to facilitate the installation, the image collector may also be arranged on an outer wall of the implant handpiece body 1.

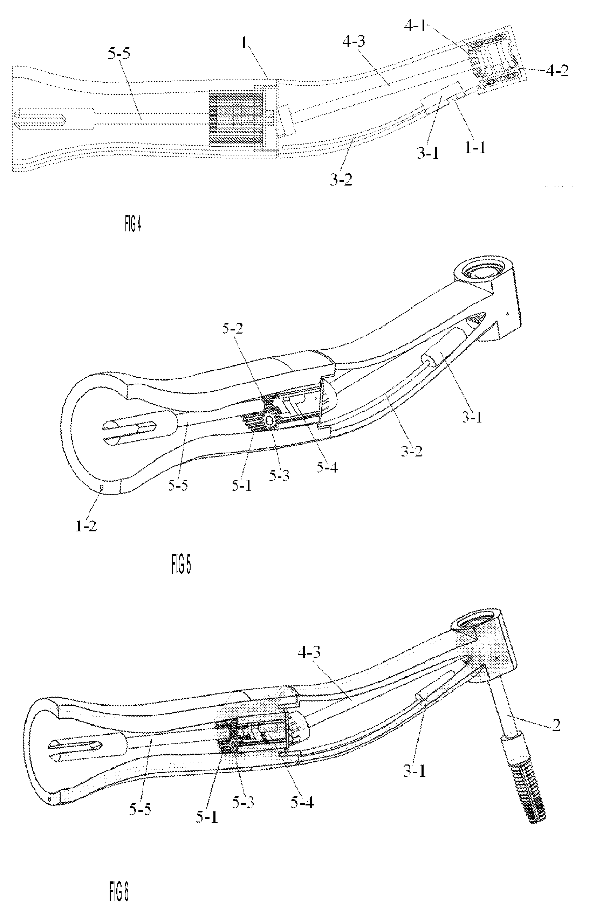

Further, as shown in FIGS. 3 to 6, a drive bevel gear 4-1, a driven bevel gear 4-2 and a reducer are provided in the inner cavity of the implant handpiece body 1, a socket is provided on a side wall of a top end of the implant handpiece body 1, a first end of the drill bit 2 is inserted in the socket and a second end of the drill bit 2 extends outward, i.e., protrudes from the implant handpiece body 1; the driven bevel gear 4-2 is disposed at the first end of the drill bit 2, the drive bevel gear 4-1 meshes with the driven bevel gear 4-2, and the drive bevel gear 4-1 is connected to the reducer through a transmission shaft 4-3. In an embodiment, an axis of the drive bevel gear 4-1 is perpendicular to an axis of the driven bevel gear 4-2 to avoid axial forces when the drive bevel gear 4-1 and the driven bevel gear 4-2 mesh with each other. Since the drive bevel gear 4-1 and the driven bevel gear 4-2 mesh with each other, when the drive bevel gear 4-1 is driven to rotate by the reducer through the transmission shaft 4-3, the driven bevel gear 4-2 will rotate with it, and thus the drill bit 2 may be driven to rotate.

Further, the reducer includes a motor, an inner ring gear 5-1, a planet carrier 5-4, and a planet gear 5-3 and a sun gear 5-2 disposed in the inner ring gear 5-1; the transmission shaft 5-3 is connected to the planet gear 5-3 through the planet carrier 5-4, the planet gear 5-3 is located between the inner ring gear 5-1 and the sun gear 5-2, and the planet gear 5-3 meshes with the inner ring gear 5-1 and the sun gear 5-2, respectively; the sun gear 5-2 is connected to the motor through a rotating shaft 5-5. Since the sun gear 5-2 and the planet gear 5-3 mesh with each other, when the sun gear 5-2 is driven to rotate by the motor through the rotating shaft 5-5, the planet gear 5-3 will rotate together under the drive of the sun gear 5-2 and then drive the planet carrier 5-4 to rotate, which in turn may drive the drive bevel gear 4-1 to rotate through the transmission shaft 4-3.

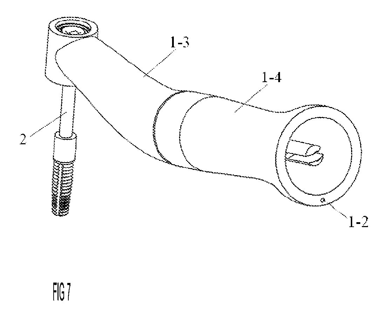

In an embodiment, as shown in FIG. 7, the implant handpiece body 1 includes a handle 1-4 and a head 1-3, one end of the head 1-3 is connected to the handle 1-4, and the other end of the head 1-3 is provided with the drill bit 2; an axis of the drill bit 2 is perpendicular to an axis of the head 1-3. Further, in order to make the dental implant handpiece more convenient to be applied to a robot, the axis of the head 1-3 is collinear with an axis of the handle 1-4. The advantages of such a configuration lie in that, on the one hand, the variables in the formula for calculating the position coordinates of the end of the drill bit 2 can be reduced so that the horizontal coordinate of the end of the drill bit 2 can be determined by only one variable, which is the length of the drill bit 2, and so that the vertical coordinate of the end of the drill bit 2 can be determined by only two variables, which are the length of handle 1-4 and the length of head 1-3, thereby greatly reducing the difficulty of positioning the end of the drill bit 2; on the other hand, the difficulty of processing the entire dental implant handpiece is reduced.

Finally, it should be noted that the embodiments above are only for illustrating the technical solutions of the present application, rather than limiting them; although the present application has been described in detail with reference to the foregoing embodiments, those skilled in the art should understand that the technical solutions documented in the foregoing embodiments may still be modified, or parts of the technical features thereof may be equivalently substituted; and such modifications or substitutions do not separate the essence of the corresponding technical solutions from the spirit and scope of the technical solutions of the embodiments of the present application.

## Claims

1. A dental implant handpiece, comprising an implant handpiece body, and a drill bit disposed on the implant handpiece body; **characterized in that** the implant handpiece body is further provided with an image collector disposed close to the drill bit and configured to acquire images of areas in the vicinity of the drill bit in an oral cavity.

2. The dental implant handpiece of claim 1, **characterized in that** the image collector is disposed in an inner cavity of the implant handpiece body, and the implant handpiece body is provided with a lens hole and a connection hole at two ends of the implant handpiece body, respectively; the image collector is configured to acquire images through the lens hole, and the image collector is connected to an external display device through the connection hole.

3. The dental implant handpiece of claim 1, **characterized in that** an inner cavity of the implant handpiece body is provided with a drive bevel gear, a driven bevel gear and a reducer, a socket is provided on a side wall of a top end of the implant handpiece body, a first end of the drill bit is inserted in the socket and a second end of the drill bit extends outward; the driven bevel gear is disposed at the first end of the drill bit, the drive bevel gear meshes with the driven bevel gear, and the drive bevel gear is connected to the reducer through a transmission shaft.

4. The dental implant handpiece of claim 3, **characterized in that** the reducer comprises a motor, an inner ring gear, a planet carrier, and a planet gear and a sun gear disposed in the inner ring gear; the transmission shaft is connected to the planet gear through the planet carrier, the planet gear is located between the inner ring gear and the sun gear, and the planet gear meshes with the inner ring gear and the sun gear, respectively, and the sun gear is connected to the motor through a rotating shaft.

5. The dental implant handpiece of claim 1, **characterized in that** the implant handpiece body comprises a handle and a head, one end of the head is connected to the handle, and the other end of the head is provided with the drill bit, and an axis of the drill bit is perpendicular to an axis of the head.

6. The dental implant handpiece of claim 5, **characterized in that** the axis of the head is collinear with an axis of the handle.

7. The dental implant handpiece of any one of claims 1 to 6, **characterized in that** the image collector comprises an endoscope and an optical fiber, one end of the optical fiber is connected to the endoscope, and the other end of the optical fiber is configured to be connected to an external display device.

8. The dental implant handpiece of claim 7, **characterized in that** the endoscope is an optical endoscope, fiber endoscope, electronic endoscope, CCD video endoscope or CMOS video endoscope.

9. The dental implant handpiece of any one of claims 1 to 6, **characterized in that** the image collector comprises a light source and a camera, and the light source is disposed close to the camera and configured to illuminate a shooting area of the camera.

10. The dental implant handpiece of claim 9, **characterized in that** a resolution increasing button and a resolution decreasing button respectively electrically connected to the camera are provided on a side wall of the implant handpiece body.
